Europäisches Patentamt

European Patent Office

Office européen des brevets

(19)

(11) Veröffentlichungsnummer: **0 175 115**
A1

# (12) EUROPÄISCHE PATENTANMELDUNG

(21) Anmeldenummer: 85109802.0

(22) Anmeldetag: 05.08.85

(51) Int. Cl.⁴: **C 07 C 69/24,** C 07 C 67/08,
C 11 B 9/00, A 61 K 7/46,
C 11 D 3/50, C 11 D 9/44

(30) Priorität: 13.08.84 DE 3429720

(71) Anmelder: Henkel Kommanditgesellschaft auf Aktien,
Postfach 1100 Henkelstrasse 67,
D-4000 Düsseldorf-Holthausen (DE)

(43) Veröffentlichungstag der Anmeldung: 26.03.86
Patentblatt 86/13

(72) Erfinder: Blösl, Siegfried, Dr., Itterstrasse 33,
D-4000 Düsseldorf (DE)
Erfinder: Bruns, Klaus, Prof. Dr., Notburgaweg 6,
D-4150 Krefeld (DE)
Erfinder: Schaper, Ulf Armin, Dr., Randstrasse 18a,
D-4150 Krefeld (DE)

(84) Benannte Vertragsstaaten: AT BE CH DE FR GB IT LI NL

(54) **Riechstoff.**

(57) Die neue Verbindung 2,2-Dimethylpropionsäure-3'-methylbut-3'-enylester wird aufgrund ihrer ungewöhnlichen Duftnote als Riechstoff, insbesondere für Wasch- und Reinigungsmittel, verwendet.

EP 0 175 115 A1

ACTORUM AG

0175115

Henkelstraße 67
4000 Düsseldorf, den 20.03.1985

HENKEL KGaA
ZR-FE/Patente

Dr. Ha/Br/Ku

P a t e n t a n m e l d u n g

D 7037 EP

"Riechstoff"

Trotz der großen Zahl der schon bekannten natürlichen und synthetischen Riechstoffe besteht auf den Gebieten der Parfümerie und der Aromaentwicklung nach wie vor Bedarf an neuen Riechstoffen, die entweder bisher unbekannte Geruchsnoten aufweisen oder aber teure oder schwer zugängliche Riechstoffe ersetzen können. Die Suche nach preiswerten Riechstoffen mit interessanten Geruchsnoten bildete auch die Grundlage für die vorliegende Erfindung.

Ester der 2,2-Dimethylpropionsäure (Pivalinsäure) haben bisher in der Parfümerie wenig Verwendung gefunden, da die bekannten Ester, soweit sie ausreichende Flüchtigkeit besitzen, uninteressante fruchtige Geruchsnoten aufweisen. Auch die bisher bekannt gewordenen Ester des 3-Methylbut-3-enols besitzen nur wenig attraktive Geruchsnoten. So weist beispielsweise der Valeriansäureester dieses Alkohols, der in der japanischen Patentanmeldung 56/92247 (Derwent-Referat 66783 D/37) erwähnt wird, einen ausgeprägt fruchtigen Geruch auf.

Es wurde nun gefunden, daß der Pivalinsäureester des 3-Methylbut-3-enols überraschenderweise nicht die in dieser Verbindungsklasse fast ausschließlich beobachtete Fruchtester-Note aufweist, sondern sich olfaktorisch hiervon völlig unterscheidet. Die Substanz besitzt dem-

. . .

gegenüber eine parfümistisch außerordentlich wertvolle Rosenoxid-Note mit einer Salicylat-Nuance. Derartige Geruchsnoten kommen insbesondere in Kombination ausgesprochen selten vor; zudem sind Substanzen, die diese Noten aufweisen, in der Regel verhältnismäßig teuer.

Gegenstand der Erfindung ist daher 2,2-Dimethylpropionsäure-3'-methylbut-3'-enylester und seine Verwendung als Riechstoff oder Aromastoff.

Der Ester ist leicht durch sauer katalysierte Veresterung von Pivalinsäure mit 3-Methylbut-3-enol herstellbar und bildet deshalb eine preiswerte Alternative zu parfümistisch ähnlichen, aber sehr viel schwerer zugänglichen Riechstoffen. Zusätzlich zu seiner interessanten Geruchsnote zeichnet ihn eine ungewöhnlich hohe Geruchsintensität aus. Weiterhin ist der neue Riechstoff, trotz der Estergruppierung im Molekül, außerordentlich stabil gegen Hydrolyse, so daß er vielseitig verwendet werden kann, insbesondere auch auf dem bevorzugten Anwendungsgebiet der Wasch- und Reinigungsmittel.

...

## Beispiele

### 1. Herstellung

In 50 ml Hexan wurden 10,2 g (0,1 Mol) 2,2-Dimethylpropionsäure und 20,6 g (0,24 Mol) 3-Methyl-but-3-en-1-ol vorgelegt. Nach Zugabe von 0,3 g para-Toluolsulfonsäure wurde erhitzt und das Reaktionswasser durch azeotrope Destillation ausgekreist. Die Umsetzung war nach ca. 4 Stunden beendet. Das Reaktionsgemisch wurde anschließend mit Wasser und verdünnter Natriumbicarbonat-Lösung gewaschen. Die organische Phase wurde abgetrennt und getrocknet; dann wurde nach Abziehen des Lösungsmittels das Produkt durch fraktionierte Destillation des Rückstandes gewonnen.

Ausbeute: 8,44 g (49,6 % der Theorie)

Siedepunkt: 64 $^\circ$C (23 mbar)

Brechungsindex: $n_D^{20} = 1,4241$

Signale im $^1$H-Kernresonanzspektrum:

(Lösung in $CDCl_3$)

$$\delta = 1,2 \ (s, \ 9 \ H, \ (CH_3)_3 \ )$$
$$1,78 \ (m, \ 3 \ H, \ CH_3)$$
$$2,33 \ (t, \ 2 \ H, \ -CH_2-)$$
$$4,16 \ (t, \ 2 \ H, \ -CH_2-O-)$$
$$4,72 \ (m, \ 2 \ H, \ = CH_2)$$

### 2. Verwendung

Die folgende Parfümkomposition eignet sich in besonderem Maße für Waschmittel, Seifen und Wäsche-weichspülmittel:

...

| | |
|---|---|
| 2,2-Dimethylpropionsäure-3'-methyl-but-3'-enylester | 50 Gew.-Teile |
| Irotyl ® (Henkel) | 400  "   " |
| Floramat ® (Henkel) | 200  "   " |
| Dihydromyrcenol | 100  "   " |
| Galaxolid ® (IFF) | 80  "   " |
| Jasmacylat ® (Henkel) | 70  "   " |
| Cyclohexylsalicylat | 40  "   " |
| Cumarin | 20  "   " |
| Ambroxan ® (10 % in Isopropylmyristat ) (Henkel) | 20  "   " |
| Veramoss ® (IFF) | 10  "   " |
| Methylnonylacetaldehyd | 10  "   " |

1000 Gew.-Teile

. . .

P a t e n t a n s p r ü c h e

1. 2,2-Dimethylpropionsäure-3'-methylbut-3'-enylester.

2. Verwendung von 2,2-Dimethylpropionsäure-3'-methyl-
   but-3'-enylester als Riechstoff oder Aromastoff.

3. Verwendung von 2,2-Dimethylpropionsäure-3'-methyl-
   but-3'-enylester nach Anspruch 2 in Wasch- oder
   Reinigungsmitteln.

4. Herstellung von 2,2-Dimethylpropionsäure-3'-methyl-
   but-3'-enylester durch sauer katalysierte Veresterung von 2,2-Dimethylpropionsäure mit 3-Methyl-
   but-3-en-1-ol.

| | EINSCHLÄGIGE DOKUMENTE | | EP 85109802.0 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP - A1 - 0 120 274 (HENKEL) <br><br> * Seite 2, Zeilen 8-29; Seiten 4-6 * <br><br> -- | 1-4 | C 07 C 69/24 <br> C 07 C 67/08 <br> C 11 B 9/00 <br> A 61 K 7/46 <br> C 11 D 3/50 <br> C 11 D 9/44 |
| A | GB - A - 1 288 423 (BP CHEMICALS) <br><br> * Anspruch 1 * <br><br> -- | 1 | |
| A | DE - A - 2 201 365 (L. GIVAUDAN) <br><br> * Seiten 1,4 * <br><br> ---- | 1-4 | |
| | | | RECHERCHIERTE SACHGEBIETE (Int. Cl.4) <br><br> C 07 C 69/00 |

Der vorliegende Recherchenbericht wurde für alle Patentansprüche erstellt.

| Recherchenort | Abschlußdatum der Recherche | Prüfer |
|---|---|---|
| WIEN | 19-12-1985 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82